Europäisches Patentamt

European Patent Office

Office européen des brevets

(19)

(11) Numéro de publication: **0 063 986**
A1

(12)
# DEMANDE DE BREVET EUROPEEN

(21) Numéro de dépôt: 82400645.6

(22) Date de dépôt: 08.04.82

(51) Int. Cl.³: **G 01 N 33/54,** G 01 N 33/80, G 01 N 1/28

(30) Priorité: 10.04.81 FR 8107271

(43) Date de publication de la demande: 03.11.82
Bulletin 82/44

(84) Etats contractants désignés: BE CH DE GB IT LI NL SE

(71) Demandeur: **Fella, Christian Paul, 26, Boulevard de Cimiez, F-06000 Nice (FR)**
Demandeur: **Hammou, Jean-Claude, 32, Avenue du Dauphiné, F-06000 Nice (FR)**

(72) Inventeur: **Fella, Christian Paul, 26, Boulevard de Cimiez, F-06000 Nice (FR)**
Inventeur: **Hammou, Jean-Claude, 32, Avenue du Dauphiné, F-06000 Nice (FR)**

(74) Mandataire: **Barnay, André François et al, Cabinet Barnay 80 rue Saint-Lazare, F-75009 Paris (FR)**

(54) Procédé de détection de la présence d'antigènes pneumocoque XIV dans un prélèvement de cellules humaines, et produits pour mettre en oeuvre ce procédé.

(57) L'invention permet une détection fiable et facile (par un petit laboratoire médical ou par un praticien) de la présence d'antigènes pneumocoque XIV dans un prélèvement de cellules humaines sous forme de tissu ou de cellules en suspension.

On met le prélèvement de cellules humaines (1) en présence d'anticorps (2) monospécifiques pneumocoque XIV de façon à provoquer la fixation de tels anticorps sur les antigènes éventuels du prélèvement (1). Après cette fixation on élimine, par lavage du prélèvement, les anticorps non fixés et l'on décèle optiquement la présence éventuelle d'un agent marqueur (4) avec lequel on a préalablement marqué (directement ou indirectement) les anticorps, ce marquage ayant lieu soit avant la fixation des anticorps aux antigènes soit après celle-ci et étant, dans ce dernier cas, suivi d'un autre lavage.

Evaluation pronostique des tumeurs malignes.

Procédé de détection de la présence d'antigènes pneumocoque
XIV dans un prélèvement de cellules humaines, et produits
pour mettre en oeuvre ce procédé.

La présente invention concerne la mise en évidence au niveau des cellules humaines, sous forme de tissu ou de cellules épithéliales en phase liquide, de la présence d'antigènes de spécificité pneumocoque XIV.

Les travaux des demandeurs ont montré que la présence ou l'absence d'un antigène du type précité constitue un critère d'évaluation pronostique des tumeurs malignes. C'est à la surface des cellules de tissu humain, ou sur les cellules de prélèvements en suspension liquide que l'on recherche cet antigène.

La présente invention a pour but de parvenir à un procédé de détection de la présence d'antigènes pneumocoque XIV dans un prélèvement de cellules humaines, c'est-à-dire soit sur une coupe histologique soit sur des cellules humaines isolées dans un milieu liquide, ce procédé devant pouvoir être mis en oeuvre non pas seulement par un chercheur mais aussi par un laboratoire médical standard, ou par un praticien ayant besoin d'un résultat immédiat et fiable lui permettant d'établir un diagnostic sûr et de préconiser dans les meilleurs délais une thérapeutique appropriée. L'invention a également pour but de parvenir à des produits permettant une mise en oeuvre facile et fiable du procédé.

Selon l'invention, le premier but précité est atteint par le fait qu'on met un prélèvement de cellules humaines en présence d'anticorps monospécifiques pneumocoque XIV de façon à provoquer la fixation de tels anticorps sur les antigènes éventuels du prélèvement puis, après écoulement du temps nécessaire à cette fixation (temps d'exposition), on élimine,par lavage du prélèvement, les anticorps non fixés sur ce prélèvement et l'on décèle optiquement la présence éventuelle d'un agent marqueur avec lequel on a préalablement marqué - directement ou indirectement - les anticorps, ce marquage ayant lieu soit avant ladite fixation des anticorps aux antigènes éventuels

soit après celle-ci et étant, dans ce dernier cas, suivi d'un autre lavage.

Considéré sous un aspect schématique, le procédé selon l'invention, tel qu'il est énoncé ci-avant, peut être présenté sous la forme d'une gamme opératoire au sein de laquelle on trouvera toujours,

a) un marquage (direct ou indirect) d'un anticorps monospécifique pneumocoque XIV,

b) une exposition du prélèvement aux anticorps monospécifiques pneumocoque XIV,

c) au moins un lavage, et

d) une détection de la présence éventuelle de l'agent marqueur, cet ensemble d'opérations intervenant soit dans l'ordre a), b), c), d), soit dans l'ordre b), c), a), c), d), cela sans exclure (dans l'un et l'autre cas) l'interposition d'opérations annexes supplémentaires telles qu'apport d'un tampon approprié, mise en suspension du prélèvement, centrifugation, filtrage, etc., comme exposé plus loin.

Il est clair que si l'on décèle la présence de l'agent marqueur sur le prélèvement, cela signifie que le prélèvement comporte, à la surface des cellules qui le composent, des antigènes de spécificité pneumocoque XIV puisque les anticorps ont été fixés et que ces anticorps sont monospécifiques de l'antigène précité. On voit donc que le procédé selon l'invention permet d'aboutir à des conclusions sûres.

Outre cette fiabilité, le procédé selon l'invention possède aussi une grande simplicité car les opérations qu'il implique sont simples et à la portée d'un petit laboratoire médical standard, ou d'un praticien.

Ce caractère de simplicité de l'invention est encore plus évident si l'opération de marquage (opération a dans la présentation schématique indiquée plus haut) est effectuée à l'avance, par exemple dans un laboratoire industriel. Ainsi, le procédé selon l'invention peut être mis en oeuvre par plusieurs opérateurs, à savoir un opérateur éventuellement industriel préparant à l'avance les anticorps marqués, et un opérateur (petit laboratoire, praticien) qui reçoit d'une part ces anticorps marqués et d'autre part le prélèvement et exécute les autres opérations du procédé, lequel se présente à lui sous une forme encore plus simple.

Dans une forme de réalisation préférée de l'invention, l'agent marqueur est une substance fluorescente (par exemple isothiocyanate de fluorescéine) ou rhodamine (par exemple isothiocyanate de tétraméthylrhodamine), et c'est par un examen sous exposition à un rayonnement ultraviolet avec des filtres appropriés qu'on décèle optiquement la présence ou l'absence éventuelle de cet agent marqueur.

Comme déjà indiqué, le marquage de l'anticorps peut être fait directement ou indirectement. Dans ce dernier cas (marquage indirect), c'est par l'intermédiaire d'un deuxième anticorps que le marquage de l'anticorps est réalisé, on marque directement ce deuxième anticorps et on le fixe au premier anticorps. Ce deuxième anticorps est avantageusement sous la forme d'un sérum anti-immunoglobuline, lequel peut être éventuellement marqué à l'avance par un fournisseur industriel, ce qui offre de grands avantages de simplicité pour un utilisateur accomplissant les autres opérations du procédé.

Le procédé selon l'invention est non seulement fiable, simple et aisé mais aussi "flexible", c'est-à-dire capable d'être mis en oeuvre sur divers types de prélèvements : coupes histologiques ou cellules isolées en suspension dans un milieu liquide.

Si le prélèvement est une coupe histologique, la gamme opératoire comporte avantageusement un déparaffinage de cette coupe histologique, une réhydratation par des bains successifs d'alcool à titrage décroissant, puis une stabilisation par un séjour de l'ordre de quelques minutes (par exemple 10 à 15 minutes) dans l'acétone, suivie du lavage de la coupe histologique effectué d'abord dans une solution à environ 0,5 M de NaCl comportant avantageusement une faible quantité d'un détergent non ionique, puis dans au moins un bain tamponné à pH de l'ordre de 7,2 à 7,3, ensuite, de préférence après un séchage, on procède à la mise en présence de la coupe histologique et d'anticorps monospécifiques pneumocoque XIV (que l'on a marqués préalablement ou concomitamment aux opérations précédentes) et, au début de cette exposition de la coupe à l'anticorps, on ajoute une très petite quantité d'un colorant histologique (par exemple du bleu Evans), puis on procède au lavage éliminant les anticorps non fixés sur la coupe histologique, ce lavage étant de préférence effectué avec une solution tamponnée à pH d'environ 7,2 à 7,3 après quoi on sèche la coupe histologique et on la monte comme connu pour examen au miscroscope à fluorescence, et c'est par un examen sous un tel microscope que l'on décèle optiquement la présence ou l'absence de l'agent marqueur.

La gamme opératoire précitée concerne le cas où le marquage de l'anticorps a eu lieu avant sa mise en présence du prélèvement. Si ce marquage a lieu après cette mise en présence, la gamme opératoire peut alors comporter les opérations suivantes, toujours dans le cas d'une coupe histologique : déparaffinage de la coupe histologique, réhydratation, puis stabilisation par un séjour de quelques minutes (par exemple 10 à 15 minutes) dans l'acétone, puis lavage de la coupe histologique, d'abord dans une solution à environ 0,5 M de NaCl comportant avantageusement une faible quantité d'un détergent non ionique, puis dans au moins un bain tamponné à pH de l'ordre de 7,2 à 7,3, ensuite avantageusement un séchage, puis la mise en présence de la coupe histologique et des anticorps monospécifiques pneumocoque XIV après laquelle intervient - après un nouveau lavage - l'application (à la coupe histologique) d'un antisérum anti-immunoglobuline que l'on a marqué préalablement ou concomitamment aux opérations précédentes, et la suite des opérations comporte un séchage de la coupe histologique puis le montage de celle-ci pour un examen au microscope à fluorescence et enfin l'examen par voie optique de la présence ou de l'absence de l'agent marqueur.

Le prélèvement peut être constitué par des cellules humaines eh phase liquide. En d'autres termes, le prélèvement peut être constitué par des cellules humaines mises en suspension après avoir été recueillies dans des humeurs (urines, liquide d'ascite, liquide pleural, ...), ou après avoir été recueillies par divers procédés de prélèvement, ce sont alors par exemple des

- cellules d'un frottis cervico-vaginal mises en suspension dans un milieu adéquat;

- cellules de lavage bronchique recueillies après une broncoscopie;

- cellules d'expectoration mises en suspension dans un milieu adéquat, ou, d'une façon générale,

- toutes cellules, épithéliales ou non, recueillies et mises en suspension.

Pour ce type de prélèvement, le procédé selon l'invention comporte, avant la mise en présence des anticorps monospécifiques pneumocoque XIV et du prélèvement, au moins une centrifugation de la suspension de cellules humaines épithéliales pour former un "culot" ou agglomérat cellulaire. La mise en suspension des cellules s'effectue avantageusement dans une solution tamponnée à pH d'environ 7,2 à 7,3 , et la centrifugation est avantageusement suivie d'une remise en suspension du premier culot formé, de l'addition d'un détergent non ionique, puis d'une deuxième centrifugation pour former un deuxième culot cellulaire qui est celui que l'on met en présence des anticorps monospécifiques pneumocoque XIV.

La mise en évidence de l'antigène pneumocoque XIV par détection de la présence de l'agent marqueur peut être effectuée de deux façons différentes lorsque le prélèvement est constitué de cellules humaines, par exemple épithéliales, recueillies et mises en suspension comme indiqué plus haut.

Selon une première possibilité, c'est dans un appareil automatique connu sous le nom de "Cell Sorter" (appareil automatique à analyser et trier les cellules) que l'examen a lieu. Dans ce cas, la mise en présence du culot cellulaire et des anticorps monospécifiques pneumocoque XIV est suivie d'une nouvelle centrifugation en phase liquide faisant office de lavage, puis d'une remise en suspension, et c'est sous la forme de la suspension ainsi obtenue que le prélèvement est examiné dans l'appareil "Cell Sorter" susmentionné.

L'autre possibilité de mise en évidence de la présence de l'antigène par détection optique de la présence de l'anticorps, dans le cas d'un prélèvement de cellules humaines mises en suspension, est le recours au microscope à fluoresence. Pour cela, la mise en présence du culot cellulaire et des anticorps monospécifiques pneumocoque XIV est suivie ou accompagnée de l'addition d'un colorant et suivie d'une remise en suspension, faisant office de lavage, dans une solution tamponnée à pH 7,2 à 7,3 que l'on filtre , de préférence sur une membrane filtrante, après quoi on monte le filtrat en tant que préparation pour examen microscopique, et l'on examine au microscope à fluorescence ce filtrat que l'on a avantageusement laissé sur la membrane filtrante. Cette membrane filtrante est avantageusement un filtre connu sous le nom de "Millipore".

Le mot "antisérum" qui est utilisé dans le présent texte et dans les revendications sert à désigner, d'une façon générale, des anticorps - quelle que soit leur origine: homme, animal, cultures cellulaires, etc. - que l'homme de l'art sait préparer et qui sont utilisés en suspension.

L'invention a également pour objets des produits, encore inconnus comme produits de fabrication systématique pour mettre en oeuvre le nouveau procédé dans l'une ou l'autre des modalités envisagées. Pour la mise en oeuvre du procédé dans les modalités avec marquage préalable et direct des anticorps, l'invention prévoit un récipient contenant un antisérum monospécifique pneumocoque XIV marqué par une substance fluorescente telle que isothiocyanate de tétraméthylrhodamine ou isothiocyanate de fluorescéine. Cet antisérum étant éventuellement accompagné par un colorant histologique tel que le bleu Evans en quantité au moins suffisante pour obtenir un renforcement avantageux du contraste optique. Ce colorant dilué, non indispensable, peut être contenu dans un autre récipient. Eventuellement, il peut être contenu dans le même récipient que l'antisérum qu'il accompagne.

Pour mettre en oeuvre le procédé dans les modalités avec marquage indirect, l'invention prévoit un jeu de produits comportant : un premier récipient contenant un antisérum anti-immunoglobuline marqué par un agent fluorescent tel que l'isothiocyanate de tétraméthylrhodamine ou l'isothiocyanate de fluorescéine, cet antisérum étant éventuellement accompagné par un colorant histologique tel que le bleu Evans en quantité appropriée comme plus haut et un deuxième récipient contenant un antisérum monspécifique pneumocoque XIV. Le colorant histologique dilué peut être contenu dans un autre récipient adjoint aux deux autres.

Eventuellement, il peut être contenu dans le même récipient que l'antisérum qu'il accompagne.

Il est à noter que dans tous les cas un apport de bleu Evans excédant la quantité précitée n'est pas préjudiciable.

De tels produits peuvent être vendus sous forme de jeux ou ensembles de produits comportant en outre au moins une seringue pour filtration et une membrane filtrante y adaptable (pouvant par exemple être montée sur la sortie de cette seringue).

Les caractéristiques et avantages de l'invention apparaîtront plus complètement à la lecture de quelques exemples non limitatifs décrits ci-après.

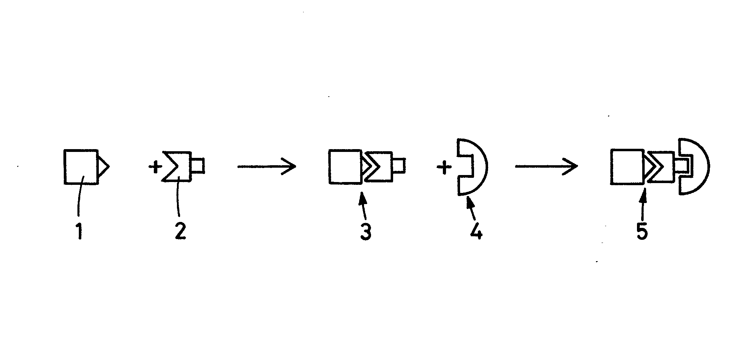

Pour commencer, l'invention sera expliquée schématiquement en se référant à la figure unique du dessin annexé illustrant les phénomènes provoqués par la mise en oeuvre de l'invention.

La référence 1 désigne une cellule avec son antigène, tandis que la référence 2 désigne un anticorps monospécifique contenu dans un antisérum monospécifique pneumocoque XIV, pas encore marqué, mis en présence de cette cellule 1. La référence 3 désigne le corps résultant de la fixation de l'anticorps 2 sur la cellule 1. La référence 4 désigne un marqueur, par exemple une anti-immunoglobuline marquée mise en présence du corps 3, c'est-à-dire mise en présence de la cellule 1 (prélèvement) après fixation de l'anticorps 2. La référence 5 désigne le corps complexe résultant de la fixation de l'anti-immunoglobuline marquée sur le corps 3. On comprend que si, après élimination complète (par lavage) de tous les éléments 2 et 4 non fixés, l'examen optique révèle la présence d'éléments 4 fixés, cela indique la présence d'une cellule avec antigène, 1.

Si l'on opère au moyen d'un antisérum déjà marqué, c'est alors un corps composé, formé par la fixation d'un marqueur 4 à un anticorps 2 (antisérum) que l'on met en présence de la cellule avec antigène, 1, pour obtenir finalement le corps complexe 5 identifiable optiquement.

Exemple 1

Pour mettre en évidence la présence (ou l'absence) de l'antigène de spécificité pneumocoque XIV sur une préparation histologique, on opère comme suit :

i) D'abord, en opérant selon les techniques habituelles connues, on confectionne une préparation histologique déparaffinée, non colorée. Ensuite, la préparation est avantageusement trempée dans des bains successifs d'alcool à titrage décroissant, puis stabilisée dans un bain d'acétone pendant 10 à 15 minutes après quoi on la lave comme suit : dans un premier temps on lave la lame dans une solution à 0,5 mole de NaCl et 0,1% d'un détergent non ionique (par exemple le produit commercialisé sous le nom de Tween, ou tout autre produit équivalent), pendant 15 minutes environ. Dans un deuxième temps, on passe la lame dans trois bains différents, avantageusement tamponnés à pH 7,2-7,3 (un tampon phosphate commercialisé sous le nom de P.B.S. convient parfaitement pour cela). La durée totale de ce lavage est de 15 minutes.

ii) Par ailleurs on a marqué, préalablement ou concomitamment, un antisérum monospécifique pneumocoque XIV, ce marquage étant effectué en lui apportant une substance fluorescente tel que l'isothiocyanate de tétraméthylrhodamine ou l'isothiocyanate de fluorescéine.

iii) Après séchage de la lame (c'est-à-dire de la préparation histologique), on la place dans une boîte métallique, à plat sur un porte-objet et on la recouvre

9

avec l'antisérum marqué (quelques gouttes suffisent). La durée de l'exposition à l'antisérum peut atteindre 30 min. (voire davantage sans que cela puisse nuire à la réaction). Au début de l'exposition, on ajoute quelques gouttes de solution à 1/1000 de bleu Evans (colorant histologique).

iv) Après cela, on lave la préparation, de préférence dans plusieurs bains successifs de solution tamponnée à pH 7,3 (5 minutes à chaque fois).

v) Ensuite, on sèche la préparation histologique que l'on monte de façon connue avec une goutte de glycérol placée sur une lamelle qui est collée sur la préparation.

vi) On exécute ensuite une lecture au microscope sous éclairage correspondant au type du marqueur, c'est-à-dire sous éclairage UV du microscope à fluorescence, avec filtre approprié, selon un mode opératoire connu.

Exemple 2

Le prélèvement est une coupe histologique et l'on exécute les opérations i) de l'exemple 1, puis on fait sécher la lame, on la place dans une boîte métallique à plat sur un porte-objet et on la recouvre d'antisérum monospécifique pneumocoque XIV non marqué (quelques gouttes suffisent). La durée de l'exposition peut atteindre 30 min. ou plus.

On lave ensuite la préparation, puis, pour marquer indirectement les anticorps de l'antisérum fixés au prélèvement , on applique un sérum anti-immunoglobuline que l'on a préparé préalablement ou concomitamment aux opérations précédentes. Cette opération peut aussi être considérée comme une deuxième exposition.

Ensuite on exécute les opérations iv), v) et vi) de l'exemple 1.

Exemple 3

Pour mettre en évidence la présence ou l'absence de l'antigène de spécificité pneumocoque XIV sur un prélèvement constitué par des cellules humaines recueillies et mises en suspension, on opère comme suit :

i) Dans une première phase, les cellules sont recueillies sur un milieu liquide fixateur et stabilisateur connu en soi comme tel.

Dans une deuxième phase, on centrifuge la suspension cellulaire ainsi obtenue (par exemple pendant 5 min. à 5000 tr/min). On récupère le culot cellulaire dans un milieu liquide comprenant un tampon phosphate de type PBS (90%) associé à un détergent non ionique (10%) tel que, par exemple, le produit commercialisé sous le nom de Tween.

Dans une troisième phase, on centrifuge à nouveau la suspension cellulaire ainsi obtenue et l'on récupère le "culot" (c'est-à-dire l'agglomérat de cellules résultant de la centrifugation).

ii) Par ailleurs, on a effectué l'opération ii) de l'exemple 1.

iii) On ajoute au culot obtenu après centrifugation l'antisérum marqué. Cette addition est faite de préférence en volume égal à celui du culot.

iv) Après mise en présence du prélèvement et de l'antisérum et maintien (exposition) pendant 10 à 30 min. (ou plus), on centrifuge de nouveau la suspension cellulaire dans un tampon phosphate de type PBS, cette opération constituant un lavage et formant un nouveau "culot" cellulaire. On recueille ce culot cellulaire et on le remet en suspension dans un tampon de type PBS, puis cette suspension (ou un échantillon de celle-ci) est passée à la machine connue sous le nom de "Cell Sorter" (appareil automatique connu, pour l'analyse et le classement des cellules), et cette machine exécute la détection finale.

Exemple 4

Il s'agit encore d'un prélèvement de cellules mises en suspension. Toutefois, c'est au microscope à fluorescence que la détection finale doit être faite.

On exécute les opérations i) à iii) de l'exemple 3 et, lors de l'opération iii, on ajoute un colorant connu (bleu Evans) à l'antisérum.

Après maintien en présence du prélèvement et de l'antisérum, on élimine l'excès d'antisérum et de colorant en procédant à un lavage qui s'effectue par centrifugation dans une solution tampon de type PBS.

Le culot formé est ensuite remis en suspension dans 5 cm$^3$ d'un tampon phosphate (PBS). Cette suspension est filtrée sur un filtre connu sour le nom de "Millipore". Pour ce filtrage, il est avantageux de placer la suspension dans une seringue avec laquelle on force le milieu liquide à traverser ce filtre connu qui est une membrane en matière plastique munie de pores dont le calibre maximal est en rapport avec la taille des cellules du prélèvement (ce filtre est donc un filtre sélectif).

Avec du glycérol, on colle ensuite, sur une lame de verre, la membrane filtrante (Millipore) portant les cellules retenues. Ensuite, on applique une lamelle pour examen microscopique, en interposant une goutte de glycérol entre le Millipore et cette lamelle.

On procède ensuite à une lecture au microscope à fluorescence.

Comme on le voit, lorsqu'il s'agit d'un prélèvement de cellules, par exemple épithéliales, mises en suspension, le lavage peut être réalisé en effectuant une mise en suspension, puis une centrifugation (en milieu liquide), ce qui permet de récupérer un "culot" de cellules lavées. Bien entendu, l'addition d'un adjuvant détergent (Tween par exemple) avant la centrifugation peut être avantageuse.

L'invention admet de nombreuses variantes de mise en oeuvre que l'homme de l'art pourra imaginer à la lumière des explications et des exemples présentés.

REVENDICATIONS

1.- Procédé de détection de la présence ou de l'absence d'antigènes de spécificité pneumocoque XIV dans un prélèvement de cellules humaines, caractérisé par le fait qu'on met le prélèvement en présence d'anticorps monospécifiques pneumocoque XIV de façon à provoquer la fixation de tels anticorps sur les antigènes éventuels du prélèvement, puis après écoulement du temps nécessaire à cette fixation (temps d'exposition), on élimine - par lavage du prélèvement - les anticorps non fixés sur ce prélèvement et l'on décèle optiquement la présence éventuelle d'un agent marqueur avec lequel on a préalablement marqué - directement ou indirectement - les anticorps, ce marquage ayant lieu soit avant ladite fixation des anticorps aux antigènes éventuels, soit après celle-ci et étant, dans ce dernier cas, suivi d'un autre lavage.

2.- Procédé selon la revendication 1, caractérisé par le fait que l'agent marqueur est une substance fluorescente telle que fluorescéine ou rhodamine, et que c'est par un examen sous exposition à un rayonnement ultraviolet que l'on décèle optiquement la présence ou l'absence éventuelle de cet agent marqueur.

3.- Procédé selon la revendication 1 ou 2, caractérisé par le fait que le marquage des anticorps est effectué indirectement, à savoir par l'intermédiaire de deuxièmes anticorps que l'on marque directement et que l'on fixe aux premiers anticorps, ces deuxièmes anticorps étant sous la forme d'un sérum anti-immunoglobuline.

4.- Procédé selon l'une quelconque des revendications précédentes, caractérisé par le fait que le marquage des anticorps est fait avant mise en présence du prélèvement et des anticorps monospécifiques pneumocoque XIV.

5.- Procédé selon l'une quelconque des revendications 1 à 3, caractérisé par le fait que le marquage par l'agent marqueur est effectué après le lavage succédant à la mise en présence du prélèvement et des anticorps monospécifiques pneumocoque XIV, et ce marquage est lui-même suivi d'un autre lavage effectué avant l'opération de détection optique.

6.- Procédé selon l'une quelconque des revendications 1 à 4, caractérisé par le fait que le prélèvement est une coupe histologique que l'on déparaffine, réhydrate, puis stabilise par un séjour dans l'acétone suivie d'un lavage de la coupe histologique effectué d'abord dans une solution à environ 0,5 M de NaCl comportant avantageusement une faible quantité d'un détergent non ionique, puis effectué dans au moins un bain tamponné à pH de l'ordre de 7,2 à 7,3, par le fait que, de préférence après un séchage, on procède à la mise en présence de la coupe histologique et d'anticorps monospécifiques pneumocoque XIV que l'on a marqué préalablement ou concomitamment aux opérations précédentes, et par le fait que, au plus tard au début de cette exposition de la coupe à l'antisérum, on ajoute de préférence une très petite quantité d'un colorant histologique, après quoi on procède au lavage éliminant les anticorps non fixés à la coupe histologique, ce lavage étant de préférence effectué avec une solution tamponnée à pH d'environ 7,2 à 7,3, après quoi on sèche la coupe histologique et on la monte comme connu pour examen au microscope à fluorescence, et c'est par un examen sous un tel microscope que l'on décèle optiquement la présence ou l'absence de l'agent marqueur.

7.- Procédé selon les revendications 1, 3 et 5, caractérisé par le fait que le pélèvement est une coupe histologique que l'on déparaffine, réhydrate, puis stabilise par un séjour dans l'acétone suivi d'un lavage de cette coupe histologique effectué d'abord dans une solution à environ 0,5 M de NaCl comportant avantageusement une faible quantité d'un détergent non ionique, puis dans au moins un bain tamponné à pH de l'ordre de 7,2 à 7,3, après quoi on exécute la mise en présence de la coupe histologique et des anticorps monospécifiques pneumocoque XIV après laquelle intervient - après un nouveau lavage - l'application (à la coupe histologique) d'un antisérum anti-immunoglobuline que l'on a marqué préalablement ou concomitamment aux opérations précédentes, et, après un séchage de la coupe histologique, on monte celle-ci pour un examen au microscope à fluorescence, et c'est par un examen sous un tel microscope que l'on décèle optiquement la présence ou l'absence de l'agent marqueur.

8.- Procédé selon l'une quelconque des revendications 1 à 5, caractérisé par le fait que le prélèvement est constitué par des cellules humaines que l'on met en suspension, après quoi on centrifuge pour former un culot ou amas cellulaire que l'on extrait pour le mettre en présence des anticorps monospécifiques pneumocoque XIV, les opérations de lavage étant faites sous la forme d'une mise en suspension suivie d'une centrifugation dans un milieu liquide que l'on sépare ensuite du culot formé par la centrifugation.

9.- Procédé selon la revendication 8, caractérisé par le fait que la mise en suspension initiale a lieu dans une solution tamponnée à pH d'environ 7,2 à 7,3 et que la centrifugation initiales est suivie d'une remise en suspension du premier culot formé, d'une addition d'un détergent non-ionique, puis d'une deuxième centrifugation pour former un deuxième culot cellulaire qui est celui que l'on met en présence des anticorps monospécifiques pneumocoque XIV.

10.- Procédé selon la revendication 8 ou 9, caractérisé par le fait que la mise en présence du culot cellulaire et des anticorps monospécifiques pneumocoque XIV est suivie d'une nouvelle centrifugation en phase liquide, faisant office de lavage, puis d'une remise en suspension et que c'est sous la forme de la suspension ainsi obtenue que le prélèvement est examiné optiquement dans un appareil automatique connu sous le nom de "Cell Sorter".

11.- Procédé selon la revendication 8 ou 9, caractérisé par le fait que la mise en présence du culot cellulaire et des anticorps monospécifiques pneumocoque XIV est suivie ou accompagnée de la mise en présence d'un colorant ajouté, et suivie d'une remise en suspension faisant office de lavage dans une solution tamponnée à pH d'environ 7,2 à 7,3 que l'on filtre de préférence sur une membrane filtrante, après quoi on monte le filtrat en tant que préparation pour examen microscopique et l'on examine, au microscope à fluorescence, ce filtrat cellulaire que l'on a avantageusement laissé sur la membrane filtrante.

12.- Produit pour mettre en oeuvre le procédé selon l'une quelconque des revendications précédentes, caractérisé par le fait qu'il comporte un récipient contenant un antisérum monospécifique pneumocoque XIV et par le fait que cet antisérum est marqué par une substance fluorescente telle qu'isothiocyanate de tétraméthylrhodamine ou isothiocyanate de fluorescéine, cet antisérum étant éventuellement accompagné par un colorant histologique tel que le bleu Evans en quantité susceptible de renforcer le contraste optique.

13.- Jeu de produits pour mettre en oeuvre le procédé selon l'une quelconque des revendications 1 à 11, caractérisé par le fait qu'il comporte un premier récipient contenant un antisérum monospécifique pneumocoque XIV et un deuxième récipient contenant un antisérum anti-immunoglobuline marqué par un agent fluorescent tel qu'isothiocyanate de tétraméthylrhodamine ou isothiocyanate de fluorescéine, cet antisérum étant éventuellement accompagné par un colorant histologique tel que le bleu Evans en quantité susceptible de provoquer le renforcement du contraste optique.

14.- Jeu de produits selon l'une quelconque des revendications 12 ou 13, caractérisé par le fait que le colorant histologique est contenu dans un autre récipient, lequel est accompagné par au moins une seringue et au moins une membrane filtrante montée, ou apte à être montée, sur la sortie de cette seringue.

15.- Produits selon l'une quelconque des revendications 12, 13 ou 14, caractérisés par le fait que le colorant histologique est contenu dans le récipient de l'antisérum qu'il accompagne.

0063986

Office européen des brevets

**RAPPORT DE RECHERCHE EUROPEENNE**

Numéro de la demande

EP 82 40 0645

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| P,X | EP-A-0 038 738 (HAMMOU et al.) * en entier * | 1-5 | G 01 N 33/54<br>G 01 N 33/80<br>G 01 N 1/28 |
| Y | CHEMICAL ABSTRACTS, vol.79, no.1, 9 juillet 1973, page 326, résumé no.3648j, Columbus, Ohio (US) H. SCHULTZE-MOSGAU et al.: "Detection of specific blood-group-receptors in the placenta with agglutinins from plants (lectins) and snails (protectins)" & Z. Immunitaetsforsch., Exp. Klin. Immunol. 1972, 144 (5), 482-90 * en entier * | 1-3 | |
| Y | FR-A-1 602 301 (STERWIM A.G.) * page 4, lignes 8-43; page 5, lignes 1-44; revendications 1-2 * | 1-3,12 | |
| A | CHEMICAL ABSTRACTS, vol.90, no.15, 9 avril 1979, page 466, résumé no.119465d, Columbus, Ohio (US) R. MAGOUS et al.: "Immunosorbent method for the detection of A.B.O. blood group specificity on CEA preparations" & Biochem Biophys Res. Commun. 1978, 85(4), 1453-9* en entier * | 1 | |

**DOMAINES TECHNIQUES RECHERCHES (Int. Cl. ³)**

G 01 N 33/00
G 01 N 1/00

Le présent rapport de recherche a été établi pour toutes les revendications

| Lieu de la recherche | Date d'achèvement de la recherche | Examinateur |
|---|---|---|
| LA HAYE | 21-07-1982 | DE LUCA G.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

0063986

Office européen
des brevets

**RAPPORT DE RECHERCHE EUROPEENNE**

Numéro de la demande

EP 82 40 0645

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl. ³) |
|---|---|---|---|
| A | CHEMICAL ABSTRACTS, vol.79, no.3, 23 juillet 1973, page 298, résumé no.16693v, Columbus, Ohio (US) J.D. COONROD et al.: "Detection of type-specific pneumococcal antigens by counterimmunoelectrophoresis, I. Methodology and immunologic properties of pneumococcal antigens" & J. Lab. Clin. Med. 1973, 81(5), 770-7 * en entier * | 1 | |
| A | GB-A-2 018 986 (SYVA COMPANY) * abrégé; page 8, ligne 40; revendications 1,16 * & FR - A - 2 434 201 & FR - A - 2 435 716 | 1-5 | |
| A | DE-A-2 744 835 (MOCHIDA SEIYAKU) * revendications 1-8; pages 6-11 * | 1-5 | DOMAINES TECHNIQUES RECHERCHES (Int. Cl. ³) |

Le présent rapport de recherche a été établi pour toutes les revendications

| Lieu de la recherche | Date d'achèvement de la recherche | Examinateur |
|---|---|---|
| LA HAYE | 21-07-1982 | DE LUCA G.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

_____

& : membre de la même famille, document correspondant

OEB Form 1503. 03.82